# EUROPÄISCHE PATENTSCHRIFT

(11) **EP 0 600 931 B1**
(45) Veröffentlichungstag und Bekanntmachung des Hinweises auf die Patenterteilung: **13.03.1996**
(21) Anmeldenummer: 92916564.5
(22) Anmeldetag: 05.08.1992
(51) Int. Cl.: A61K 7/027, A61K 7/48

(54) **KOSMETISCHE STIFTE**
COSMETIC STICKS
BATONS COSMETIQUES

(30) Priorität: 29.08.1991 DE 4128748
(43) Veröffentlichungstag der Anmeldung: 15.06.1994
(73) Patentinhaber: Beiersdorf Aktiengesellschaft, D-20245 Hamburg (DE)
(72) Erfinder: AUL, Marta, D-2110 Buchholz (DE); KLIER, Manfred, D-2055 Aumühle (DE); SCHNEIDER, Günther, D-2000 Hamburg 20 (DE); TEICHMANN, Stephan, Meguro-ku, Tokyo 152 (JP)
(86) Internationale Anmeldenummer: DE9200649
(87) Internationale Veröffentlichungsnummer: WO9304658

(56) Entgegenhaltungen:
- EP-A- 0 013 755
- EP-A- 0 014 509
- EP-A- 0 347 664
- FR-A- 1 255 602
- FR-A- 2 417 980
- GB-A- 2 079 601
- US-A- 4 894 224
- Patent Abstracts of Japan, Band 7, Nr. 285 (C-201)(1430), 20. Dezember 1983, & JP,A,58162510 (SHINEI KAGAKU K.K.) 27. September 1983, siehe Zusammenfassung & Chemical Abstracts, Band 100, Nr. 2, Januar 1984, (Columbus, Ohio, US), siehe Seite 353, Zusammenfassung Nr. 12459c, & JP,A,58162510 (SHINEI KAGAKU K.K.) 27. September 1983
- H. JANISTYN: "Handbuch der Kosmetika und Riechstoffe", 2. Auflage, Band 3: "Die Körperpflegemittel", 1973, Hüthig Verlag, Heidelberg, DE, siehe Seite 879, Beispiel 2

## Beschreibung

Die vorliegende Erfindung betrifft kosmetische Stifte, insbesondere Lippenstifte, bevorzugt Lippenpflegestifte, sowie Zusammensetzungen und Verfahren zur Herstellung kosmetischer Stifte.

Die Haut der Lippen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegestiften. Diese Produkte enthalten meist zu einem hohen Anteil Wachse und Fettkomponenten, die nach dem Auftragen eine abdeckende Schicht über den Lippen ausbilden.

In die Zubereitungen für Lippenpflegestifte können zusätzlich Wirkstoffe eingearbeitet werden, die der Lippenpflege oder dem Lippenschutz förderlich sind, z.B. Vitamine, Feuchtigkeit spendende Mittel, Lichtschutzmittel, abdeckende Pigmente usw.

Die Lederhaut der Lippen weist gut durchblutete Papillen auf, die bis dicht unter die Lippenoberfläche reichen. Daher sind die Lippen rötlich gefärbt und, je nach Teintfarbe der betreffenden Person, von der übrigen Gesichtshaut mehr oder weniger stark farblich abgesetzt. Ein Stilmittel der dekorativen Kosmetik ist dann auch, die Lippenfarbe durch entsprechende Kosmetika auf den Typ der Person abzustimmen.

Produkte dieser Art sind dekorative Lippenstifte, in welche verschiedenste Farbpigmente eingearbeitet werden können. Auch diese Stifte enthalten zu hohen Anteilen Wachse und Fettkomponenten, die nach dem Auftragen eine abdeckende Lipidschicht über den Lippen ausbilden.

Die Aufgabe dieser Schicht ist jedoch nicht vorderhand, die Lippenhaut vor dem Austrocknen zu schützen. Die Lipidschicht dient hier als auf den Lippen haftende Grundlage für die eingearbeiteten Pigmentstoffe; die Pigmente selbst können aus mancherlei Gründen nicht ohne eine solche Grundlage auf die Lippen aufgetragen werden.

Es ist auch möglich, die Eigenschaften der pflegenden und dekorativen Lippenstifte miteinander zu kombinieren, d.h., in dekorative Lippenstifte pflegende oder schützende Substanzen einzuarbeiten.

Technisch betrachtet sind fast alle Lippenstifte wasserfreie Fettmischungen aus festen oder halbfesten Wachsen und flüssigen Ölen, wobei die hochgereinigten Paraffinöle und - wachse die Lippenstiftgrundmasse darstellen.

Nach dem idealen Anforderungsprofil sollen sich Lippenstifte glatt und ohne großen Reibungswiderstand auftragen lassen. Außerdem soll ein Lippenstift schon bei leichtem Andruck einen nicht schmierigen, stumpfen oder klebrigen, aber dennoch gut haftenden Fettfilm an die Lippen abgeben. Durch diesen Fettfilm sollen die Lippen dann glatt und geschmeidig gemacht werden.

Darüber hinaus muß ein Lippenstift auch noch die Anforderungen erfüllen, daß er bruchfest und temperaturbeständig sein muß und nicht ausölen darf.

Übliche Grundstoffe des Standes der Technik sind
(1) flüssige Öle (z.B. Paraffinöle, Ricinusöl, Isopropylmyristat)
(2) halbfeste Bestandteile (z.B. Vaseline, Lanolin)
(3) feste Bestandteile (z.B. Bienenwachs, , Ceresin und Mikrokristalline Wachse bzw. Ozokerit)
(4) hochschmelzende Wachse (z.B. Carnaubawachs, Candelillawachs)
Lippenstifte des Standes der Technik mit einem Gehalt an Paraffinen und Bienenwachs sind in "Kosmetik, Entwicklung Herstellung und Anwendung kosmetischer Mittel", S. 105 , Herausgeber: W.Umbach, Georg Thieme Verlag, Stuttgart - New York, 1988, beschrieben.

Der Stand der Technik hat aber eine Reihe von Nachteilen. Dazu zählt die Tatsache, daß wasserlösliche Wirkstoffe häufig nicht gut genug fettlöslich sind, als daß sie in nennenswertem Maße in die kosmetischen Grundlagen einzubauen wären. Andererseits wäre ein gewisser Wassergehalt durchaus erwünscht, um die Kompatibilität des kosmetischen Stiftes mit der menschlichen Haut zu erhöhen.

So ist aus dem DBP 23 35 549 ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel herstellt, dieses mit einer kosmetischen Grundlage vermischt und dann einen Gehalt an Wasser in die Mischung emulgiert.

Nach diesem Verfahren sind jedoch keine Stifte herzustellen, die über die gestellten universellen Anforderungen an einen kosmetischen Stift verfügen.

Ein weiterer Nachteil ist, daß Paraffinöle und -wachse bis zum gegenwärtigen Zeitpunkte unabdingbare Bestandteile für Lippenstifte waren. Obwohl es sich dabei um in guter Qualität erhältliche Rohstoffe handelt, und Stifte mit brauchbaren Eigenschaften mit ihrer Hilfe formuliert werden können, sind die Anwendungseigenschaften solcher kosmetischer Stifte jedoch begrenzt. Außerdem sind Paraffine wertvolle Grundstoffe, deren Vorkommen auf der Erde begrenzt sind. Die moderne Produktion geht in die Richtung nachwachsender Rohstoffe, also beispielsweise pflanzlicher Wachse oder Öle auf dem Gebiete der Kosmetik.

Es war jedoch bisher unmöglich, einen kosmetischen Stift auf der Basis der bekannten pflanzlichen Wachse, Fette oder Öle oder chemisch modifizierter pflanzlicher Wachse, Fette oder Öle zu konzipieren. Eine weitere Aufgabe der vorliegenden Erfindung war also, eine Grundlage für kosmetische Stifte, insbesondere Lippenstifte zu schaffen, welche auf mineralische Öle verzichten kann und stattdessen auf pflanzlichen oder gegebenenfalls tierischen Lipidkomponenten bzw. chemisch modifizierten Varianten davon basieren kann.

Es war überraschend und nicht vorhersehbar, daß kosmetische Stifte, dadurch gekennzeichnet, daß sie Emulsionen darstellen und als wesentliche Bestandteile
(a) Bienenwachs,
(b) einen oder mehrere Ester aus
   (ba) einer gesättigten Carbonsäure mit 14 - 34 Kohlenstoffatomen und
   bb) einem gesättigten Alkohol mit 20 - 40 Kohlenstoffatomen
(c) Wasser, sowie
(d) gegebenenfalls weitere Lipide und/oder übliche Hilfs- und Zusatzstoffe enthalten,
die Nachteile des Standes der Technik beseitigen würden.

Es ist insbesondere möglich, mit den erfindungsgemäßen Zusammensetzungen Emulsionslippenstifte oder -pflegestifte zu erhalten und auf einen Zusatz mineralischer Lipidkomponenten oder Paraffine gänzlich zu verzichten.

Darüberhinaus ermöglicht die vorliegende Erfindung, Emulsionslippenstifte zur Verfügung zu stellen, welche die Nachteile des Standes der Technik nicht aufweisen.

Der Stand der Technik, zu nennen sind Patent Abstracts of Japan, Vol. 7, No. 285 (C-201)[1430], bzw. Chem.Abstr. 100, 12 459c, US-A-4,894,224, EP-A-14 509, FR-A-1 255 602 und EP-A-13 755, konnte nicht den Weg zur vorliegenden Erfindung aufzeigen.

Vorteilhaft ist insbesondere, die Ester aus der Gruppe der Ester aus einer gesättigten Carbonsäure mit 14 - 23 Kohlenstoffatomen und einem gesättigten Alkohol mit 24 - 34 Kohlenstoffatomen zu wählen.

Vorteilhaft sind insbesondere die Ester aus einer gesättigten Carbonsäure mit 16 - 20 Kohlenstoffatomen und einem gesättigten Alkohol mit 26 - 32 Kohlenstoffatomen.

Ganz besonders vorteilhaft sind die Ester (A) - (J):
(A) Hexacosanylpalmitat (Synonym: Hexacosanylhexadecanoat) hat die Formel 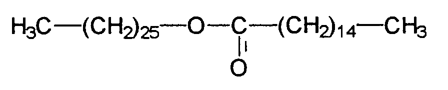
(B) Octacosanylpalmitat (Synonym: Octacosanylhexadecanoat) hat die Formel 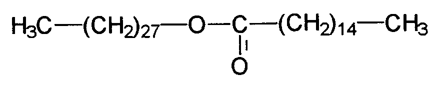
(C) Triacontanylpalmitat (Synonym: Triacontanylhexadecanoat) hat die Formel 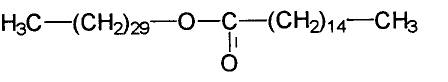
(D) Dotriacontanylpalmitat (Synonym: Dotriacontanhexadecanoat) hat die Formel 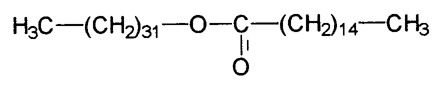
(E) Tetratriacontanylpalmitat (Synonym: Tetratriacontanhexadecanoat) hat die Formel 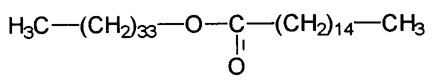
(F) Hexacosanylstearat (Synonym: Hexacosanyloctadecanoat) hat die Formel 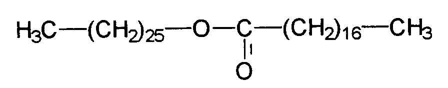
(G) Octacosanylstearat (Synonym: Octacosanyloctadecanoat) hat die Formel 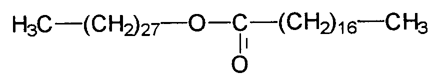
(H) Triacontanylstearat (Synonym: Triacontanyloctadecanoat) hat die Formel 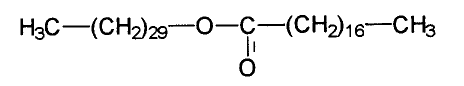
(I) Dotriacontanylstearat (Synonym: Dotriacontanoctahexadecanoat) hat die Formel 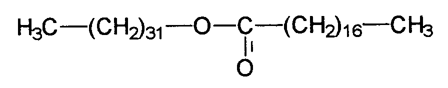
(J) Tetratriacontanylstearat (Synonym: Tetratriacontanyloctadecanoat) hat die Formel 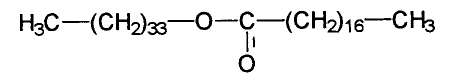
Der bevorzugte Ester ist das Octacosanylstearat.

Die Ester wurden von der Firma Koster Keunen Holland B.V. synthetisiert und zur Verfügung gestellt.

Herstellungsbedingt können die Ester gegebenenfalls gewisse unschädliche Nebenprodukte sowie nicht umgesetzte Ausgangssubstanzen enthalten. Vorteilhaft ist es, Handelsware einzusetzen, deren Gehalt an Ester mindestens 80 Gew.-%, bezogen auf das Gesamtgewicht des Produktes, enthält.

Bienenwachs (Synonyme sind: Cera flava (gelblich) und Cera alba (weiß), oder nach CTFA: Beeswax) besteht hauptsächlich aus Myricylpalmitoat, Cerotinsäure, Melissinsäure, höheren Alkoholen und Kohlenwasserstoffen. Neuere Untersuchungen legen nahe, daß Bienenwachs aus einer homologen Reihe von vornehmlich Hexadecanoatalkylestern zwischen C₃₅ und C₅₄ bestehen ("Lexikon der Hilfsstoffe für Pharmazie, Kosmetik und angrenzende Gebiete", H.P. Fiedler, 3. Aufl., 1989, Editio Cantor Aulendorf, und dort unter dem Stichwort "Bienenwachs" zitierte Quellen und Querverweise).

Bienenwachs ist seit alters ein wichtiger kosmetischer Bestandteil, insbesondere für Crèmes und Salbenzubereitungen, aber auch für kosmetische Stifte. Ein Lippenstift des Standes der Technik enthält 4,0 Gew.-% Bienenwachs.

Zu hohe Anteile an Bienenwachs haben bisher immer dazu geführt, daß kosmetische Stifte glanzlos und körnig wurden. Auch die Stabilität solcher Stifte ließ ständig zu wünschen übrig. Es war immer ein erheblicher Nachteil, daß auf Bienenwachs basierende kosmetische Stifte kosmetisch unelegant waren.

Eine gewisse Menge Bienenwachs allerdings verleiht Lippenstiften bekanntermaßen Festigkeit und bewirkt, daß die Lippenstiftmasse gut auf den Lippen haftet ("Kosmetik, Entwicklung, Herstellung und Anwendung kosmetischer Mittel", S. 104 und passim, W. Umbach (Herausgeber), 1988, Georg Thieme Verlag, Stuttgart). Gleichwohl ist Bienenwachs, wohl aus den obenstehenden Erwägungen, kein obligatorischer Bestandteil kosmetischer Stifte.

Vorteilhaft enthalten die erfindungsgemäßen kosmetischen Stifte
- 0,5 - 50 Gew.-%: Bienenwachs
- 0,5 - 50 Gew.-%: eines Esters aus einer gesättigten Carbonsäure mit 14 - 34 Kohlenstoffatomen und einem gesättigten Alkohol mit 20 - 40 Kohlenstoffatomen
- 0,1 - 20 Gew.-%: Wasser,
insbesondere
- 2,0 - 20 Gew.-%: Bienenwachs
- 2,0 - 25 Gew.-%: eines Esters aus einer gesättigten Carbonsäure mit 14 - 34 Kohlenstoffatomen und einem gesättigten Alkohol mit 20 - 40 Kohlenstoffatomen
- 1,0 - 10 Gew.-%: Wasser,
bevorzugt
- 3,0 - 10 Gew.-%: Bienenwachs
- 5,0 - 20 Gew.-%: eines Esters aus einer gesättigten Carbonsäure mit 14 - 34 Kohlenstoffatomen und einem gesättigten Alkohol mit 20 40 Kohlenstoffatomen
- 1,0 - 5 Gew.-%: Wasser.
ganz besonders bevorzugt
- 4,0 - 6,0: Gew.-% Bienenwachs
- 15,0 - 18,0 Gew.-%: eines Esters aus einer gesättigten Carbonsäure mit 14 - 34 Kohlenstoffatomen und einem gesättigten Alkohol mit 20 - 40 Kohlenstoffatomen
- 2,0 - 3,0 Gew.-%: Wasser
Es ist bevorzugt, das Verhältnis von Bienenwachs und einem oder mehreren Estern aus einer gesättigten Carbonsäure mit 14 - 34 Kohlenstoffatomen und einem gesättigten Alkohol mit 20 - 40 Kohlenstoffatomen insbesondere den Estern (A) - (J), besonders bevorzugt Octacosanylstearat, im Verhältnis von etwa 1 : 1 bis etwa 1 : 5, insbesondere etwa 1 : 3 zu wählen.

Die erfindungsgemäßen Zusammensetzungen, die als Grundlagen für Emulsionslippenstifte dienen, enthalten also eine gewisse Menge Wasser. Es ist zwar möglich, auf einen Emulgator zu verzichten, da Bienenwachs selbst Emulgatoreigenschaften hat. Vorteilhaft und bevorzugt ist jedoch, einen Emulgator einzuarbeiten, insbesondere einen Wasser-in-Öl-Emulgator (W/O-Emulgator).

Als besonders bevorzugte W/O-Emulgatoren, die zu überraschend stabilen kosmetischen Stiften mit besonders guter Hautfreundlichkeit führen und darüberhinaus kosmetisch besonders elegante Rezepturen ergeben, haben sich überraschend die Polyglycerinfettsäureester erwiesen. Besonders vorteilhaft ist das Polyglyceryl-3-diisostearat (Synonym: Triglyceryldiisostearat).

Die erfindungsgemäß vorteilhaften Polyglycerinester, deren Vorkommen, Eigenschaften und Verwendung in der Kosmetik sind aufgezählt und abgehandelt im "Lexikon der Hilfsstoffe für Pharmazie, Kosmetik und angrenzende Gebiete", H.P. Fiedler, 3. Aufl., 1989, Editio Cantor Aulendorf, dort unter den Stichwörtern "Polyglycerinester", "Polyglycerolester", "Polyglyceryl-3 diisostearate", "Triglycerin" sowie "Triglyceryldiisostearat" usw. zitierte Quellen und Querverweise. Die dort beschriebenen Polyglycerinester sind für die vorliegende Erfindung gut geeignet.

Erfindungsgemäß sind also kosmetische Stifte, welche außer durch einen Gehalt an Bienenwachs, Octacosanylstearat und Wasser dadurch gekennzeichnet sind, daß sie einen oder mehrere Emulgatoren aus der Gruppe der Polyglycerinfettsäureester, insbesondere das Polyglyceryl-3-diisostearat enthalten.

Die Emulgatoren können vorteilhaft in Gehalten von 0,05 - 10,0 Gew.-% in die erfindungsgemäßen Zusammensetzungen eingearbeitet werden, insbesondere in Gehalten von 1,00 - 8,00 Gew.-%, besonders bevorzugt 2,00 - 4,00 Gew.-%.

In die erfindungsgemäßen Zubereitungen können vorteilhaft die üblichen Bestandteile kosmetischer Stifte eingearbeitet werden, also Wachse, insbesondere pflanzliche und/oder tierische Wachse oder chemisch modifizierte Derivate davon, insbesondere Carnaubawachs, Candelillawachs und dergleichen, Kohlenwasserstoffe, Fette und Öle für die Grundsubstanz, sowie die üblichen Hilfs- und Zusatzstoffe wie Parfümöle, Konservierungsmittel, Farbpigmente, Lichtschutzmittel, Stabilisatoren.

Zusätzlich können Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Es ist insbesondere von Vorteil, den an 100 Gew.-% fehlenden Rest der Stiftmasse aus der Gruppe der folgenden Stoffe zu wählen:

Glycerintricarbonsäureester (Synonym: Triglyceride), Guerbet-Alkohole, Myristylmyristat, Jojobaöl und verwandte Substanzen. Insbesondere können vorteilhaft weitere flüssige Fettkomponenten in die erfindungsgemäßen Zusammensetzungen eingearbeitet werden, dazu zählen z.B. fraktionierte Kokosöle.

Auch hier können vorteilhaft zusätzliche Hilfs- und Zusatzstoffe sowie Wirkstoffe, insbesondere Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Erfindungsgemäß sind also auch Zusammensetzungen für kosmetische Stifte, enthaltend
(a) Bienenwachs
(b) Octacosanylstearat und
(c) Wasser
und Stoffe, gewählt aus der Gruppe
(d) Glycerintricarbonsäureester
(e) Guerbet-Alkohole
(f) Myristylmyristat
(g) Jojobaöl
(h) fraktionierte Kokosöle,
und gegebenenfalls zusätzlich Stoffen, gewählt aus der Gruppe der Wachse, Kohlenwasserstoffe, Fette, Öle, weitere Hilfs- und Zusatzstoffe wie Parfümöle, Konservierungsmittel, Farbpigmente, Lichtschutzmittel, Stabilisatoren sowie der fettlöslichen und/oder wasserlöslichen Wirkstoffe.

Es ist allerdings, aus den zuvor angegebenen Gründen vorteilhaft, auf Kohlenwasserstoffe gänzlich zu verzichten. Nichtsdestoweniger ergeben die erfindungsgemäßen Zusammensetzungen auch zusammen mit Kohlenwasserstoffen äußerst anspruchsvolle kosmetische Stifte mit vorteilhaften Eigenschaften.

Die Glycerintriester können vorteilhaft aus der Gruppe der sogenannten Neobee-Oils und der Fette gewählt werden. Zum Stande der Technik der Glycerintriëster, deren Vorkommen, Eigenschaften und Verwendung in der Kosmetik siehe "Lexikon der Hilfsstoffe für Pharmazie, Kosmetik und angrenzende Gebiete", H.P. Fiedler, 3. Aufl., 1989, Editio Cantor Aulendorf, dort unter dem Stichwort "Triglyceride" zitierte Quellen und Querverweise. Die dort beschriebenen Triglyceride sind für die vorliegende Erfindung gut geeignet.

Unter Guerbet-Alkoholen werden verzweigtkettige Alkohole des allgemeinen Schemas 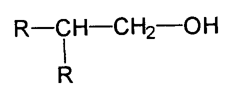
verstanden. R stellt dabei bevorzugt Kohlenwasserstoffreste von C₆ bis C₁₂ dar (Zum Stande der Technik der Herstellung, Eigenschaften und Verwendung der Guerbet-Alkohole: "Lexikon der Hilfsstoffe für Pharmazie, Kosmetik und angrenzende Gebiete", H.P. Fiedler, 3. Aufl., 1989, Editio Cantor Aulendorf, dort unter dem Stichwort "Guerbet-Alkohole" zitierte Quellen und Querverweise).

Obwohl es denkbar ist und gegebenenfalls vorteilhaft sein kann, Stifte gemäß der vorliegenden Erfindung auch für andere Anwendungen denn als Lippenstifte zu konzipieren, beispielsweise als Deodorantstifte ("Sticks"), ist in den betreffenden Anwendungsgebieten mit vergleichsweise geringer Verbraucherakzeptanz zu rechnen, da die erfindungsgemäßen Massen zumeist leicht fettig wirken. Letzteres ist bei einem Lippenstift auch durchaus erwünscht und sehr angenehm.

Von einem Deodorantstift wird jedoch, jedenfalls nach Einschätzung des mitteleuropäischen Verbraucherspektrums, erwartet, daß er eben nicht fettet.

Die erfindungsgemäßen kosmetischen Stifte lassen sich vorteilhaft erzeugen nach einem Verfahren , dadurch gekennzeichnet, daß man
(1) Bienenwachs und Octacosanylstearat, sowie gegebenenfalls einen oder mehrere Emulgatoren, gewählt aus der Gruppe der W/O-Emulgatoren, insbesondere der Polyglycerinfettsäureester, besonders vorteilhaft: Polyglyceryl-3-diisostearat
   sowie Fettkomponenten, gewählt aus der Gruppe der Glycerintricarbonsäureester Guerbet-Alkohole, Myristylmyristat, Jojobaöl und/oder verwandter Substanzen und der fraktionierten Kokosöle
aufschmilzt und gegebenenfalls den Schritten (2) und (3) unterwirft, nämlich
(2) kontinuierlich Wasser hinzugibt, bis der gewünschte Wassergehalt des kosmetischen Stiftes erreicht ist,
(3) während der Wasserzugabe die entstandene Mischung verrührt,
(4) und die gleichmäßige verrührte Mischung dann in Gußformen einbringt und langsam abkühlen läßt.

In den nachfolgenden Beispielen soll die vorliegende Erfindung beschrieben werden. Die einzelnen Einsatzstoffe sind in Analogie zur CTFA-Nomenklatur gewählt, außer bei solchen Substanzen, in welchen eine einfache chemische Bezeichnung existiert (z.B: Wasser).

### Beispiel 1

| | Gew.-% |
|---|---|
| Caprylic/Capric Triglyceride | 25 |
| Octyldodecanol | 25 |
| Caprylic/Capric Diglyceryl | |
| Succinate | 5 |
| Jojoba Öl | 5 |
| Myristylmyristat | 10 |
| Octacosanylstearat | 20 |
| Bienenwachs weiß | 9 |
| Octyl Methoxycinnamat | 1 |

### Beispiel 2

| | Gew.-% |
|---|---|
| Octyldodecanol | 45 |
| Caprylic/Capric Diglyceryl | |
| Succinate | 10 |
| Squalan | 7 |
| Jojoba Öl | 3 |
| Myristylmyristat | 8 |
| Carnauba Wachs | 2 |
| Octacosanylpalmitat | 11 |
| Octacosanylstearat | 4 |
| Beenenwachs weiß | 10 |

### Beispiel 3

| | Gew.-% |
|---|---|
| Cetylpalmitat | 8 |
| Squalan | 15 |
| Weizenkeimöl | 5 |
| Propylenglycoldicaprylat/caproat | 10 |
| Caprylic/Capric Triglyceride | 10 |
| Octyldodecanol | 20 |
| Polyisobuten | 2 |
| Cyclomethicone | 10 |
| Hexacosanylpalmitat | 10 |

### Beispiel 4

| | Gew.-% |
|---|---|
| Bienenwachs weiß | 26 |
| Caprylic/Capric Diglyceryl | |
| Succinate | 15 |
| Propylenglycol | |
| Dicaprylate/Dicaprate | 7 |
| Caprylic/Capric Triglyceride | 24 |
| Squalan | 10 |
| Schibutter | 7 |
| Octacosanylstearat | 6 |
| Octylmethoxycinnamat | 5 |

### Beispiel 5

| | Gew.-% |
|---|---|
| Caprylic/Capric Triglyceride | 14 |
| Octyldodecanol | 20 |
| Jojoba Öl | 10 |
| Myristylmyristat | 10 |
| Octacosanylstearat | 20 |
| Cetylpalmitat | 2 |
| Bienenwachs weiß | 8 |
| Polyglyceryl-3-diisostearat | 4 |
| Glycin | 1 |
| ZnSO₄ * H₂O | 1 |
| Wasser (der pH wird mit NaOH | |
| auf 6 - 7 eingestellt) | 10 |

### Beispiel 6

| | Gew.-% |
|---|---|
| Caprylic/Capric Triglyceride | 20 |
| Octyldodecanol | 15 |
| Cetearylalkohol | 5 |
| Schibutter | 10 |
| Jojoba Öl | 8 |
| Polyglyceryl-3-diisostearat | 2 |
| Polyisobuten | 2 |
| Octacosanylstearat | 10 |
| Cetylpalmitat | 5 |
| Bienenwachs weiß | 10 |
| Glycerin | 3 |
| Weizenkeimöl | 2 |
| Triacontanylpalmitat | 5 |
| Wasser | 3 |

### Beispiel 7

| | Gew.-% |
|---|---|
| Octyldodecanol | 42 |
| Caprylic/Capric Diglyceryl | |
| Succinate | 5 |
| Squalan | 5 |
| Jojoba Öl | 5 |
| Myristylmyristat | 8 |
| Octacosanyl Stearat | 20 |
| Bienenwachs weiß | 10 |
| Polyglyceryl-3-Diisostearat | 2 |
| Wasser | 3 |

### Beispiel 8

| | Gew.-% |
|---|---|
| Caprylic/Capric Triglyceride | 25 |
| Octyldodecanol | 11 |
| Caprylic/Capric Diglyceryl | |
| Succinate | 12,5 |
| Propylenglycol | |
| Dicaprylat/Dicaprat | 7 |
| Bienenwachs weiß | 26 |
| Polyglyceryl-3-Oleat | 3,5 |
| Octacosanylpalmitat | 5 |
| Wasser | 10 |

### Beispiel 9

| | Gew.-% |
|---|---|
| Jojoba Öl | 4 |
| Myristylmyristat | 7 |
| Polyglyceryl-3-oleat | 4 |
| Glyceryllanolat | 1,5 |
| Wollwachsalkohol | 1 |
| Octacosanylstearat | 5 |
| Ceresin | 15 |
| Caprylic/Capric Diglyceryl | |
| Succinate | 12,5 |
| Propylenglycol | |
| Dicaprylat/Dicaproat | 7 |
| Caprylic Capric Triglyceride | 2,5 |
| Octyldodecanol | 33 |
| Cetearylalkohol | 0,5 |
| Tocopherylacetat | 0.1 |
| Wasser | 4,9 |

### Beispiel 10

Die kosmetischen Stifte gemäß den Beispielen 1 - 9 wurden anhand subjektiver und objektiver Bewertungskriterien beurteilt.

Subjektive Bewertungskriterien waren das Gefühl beim Auftragen (stumpf, geschmeidig, fettig, schmierig, klebrig), der optische Eindruck (matt, glänzend,). In allen Fällen zeichneten sich die erfindungsgemäßen kosmetischen Stifte durch überlegenes Auftragegefühl und ausgezeichneten optischen Eindruck aus.

Objektive Bewertungskriterien waren Bruchstabilität, Masseabrieb, Penetration.

Im Vergleich mit Zusammensetzungen des Standes der Technik wiesen die erfindungsgemäßen Zusammensetzungen eine verbesserte Bruchstabilität auf.

Der Masseabrieb ist leicht zu ermitteln, indem ein Stift mit einer definierten Andruckkraft auf einer Unterlage entlanggezogen wird. Stifte mit niedrigem Masseabrieb werden als trocken, Stifte mit hohem Masseabrieb als schmierig empfunden.

Im Vergleich mit Zusammensetzungen des Standes der Technik wiesen die erfindungsgemäßen Zusammensetzungen einen verbesserten Masseabrieb auf. Die Masseabriebswerte liegen in dem Bereich, der mit einem sehr angenehmen Anwendungsgefühl verbunden wird.

Die Penetration ist zu ermitteln, indem die Eindringtiefe einer spitzen Nadel in die Lippenstiftmasse gemessen wird. Stiftmassen, bei welchen die Nadel kaum eindringt, werden als hart empfunden, solche, bei welchen die Nadel sehr leicht eindringt wird ebenfalls leicht unangenehm, weil zu weich empfunden.

Im Vergleich mit Zusammensetzungen des Standes der Technik wiesen die erfindungsgemäßen Zusammensetzungen eine verbesserte Penetration auf. Die Penentrationswerte liegen in dem Bereich, der mit einem sehr angenehmen Anwendungsgefühl verbunden wird.

## Patentansprüche

1. Kosmetische Stifte, dadurch gekennzeichnet, daß sie Emulsionen darstellen und als wesentliche Bestandteile
(a) Bienenwachs,
(b) einen oder mehrere Ester aus
(ba) einer gesättigten Carbonsäure mit 14 - 34 Kohlenstoffatomen und
(bb) einem gesättigten Alkohol mit 20 - 40 Kohlenstoffatomen
(c) Wasser, sowie
(d) gegebenenfalls weitere Lipide und/oder übliche Hilfs- und Zusatzstoffe
enthalten.

2. Kosmetische Stifte nach Anspruch 1, dadurch gekennzeichnet, daß sie
0,5 - 50 Gew.-% Bienenwachs
0,5 - 50 Gew.-% eines oder mehrerer Ester aus einer gesättigten Carbonsäure mit 14 - 34 Kohlenstoffatomen und einem gesättigten Alkohol mit 20 - 40 Kohlenstoffatomen
0,1 - 20 Gew.-% Wasser,
insbesondere
2,0 - 20 Gew.-% Bienenwachs
2,0 - 25 Gew.-% eines oder mehrerer Ester aus einer gesättigten Carbonsäure mit 14 - 34 Kohlenstoffatomen und einem gesättigten Alkohol mit 20 - 40 Kohlenstoffatomen
1,0 - 10 Gew.-% Wasser,
bevorzugt
3,0 - 10 Gew.-% Bienenwachs
5,0 - 20 Gew.-% eines oder mehrerer Ester aus einer gesättigten Carbonsäure mit 14 - 34 Kohlenstoffatomen und einem gesättigten Alkohol mit 20 - 40 Kohlenstoffatomen
1,0 - 5 Gew.-% Wasser,
ganz besonders bevorzugt
4,0 - 6,0 Gew.-% Bienenwachs
15,0 - 18,0 Gew.-% eines oder mehrerer Ester aus einer gesättigten Carbonsäure mit 14 - 34 Kohlenstoffatomen und einem gesättigten Alkohol mit 20 - 40 Kohlenstoffatomen
2,0 - 3,0 Gew.-% Wasser
enthalten.

3. Kosmetische Stifte nach Anspruch 1 oder 2, dadurch gekennzeichnet, daß sie Bienenwachs und einen oder mehrere Ester aus einer gesättigten Carbonsäure mit 14 - 34 Kohlenstoffatomen und einem gesättigten Alkohol mit 20 - 40 Kohlenstoffatomen im Verhältnis von 1 : 1 bis 1 : 5, insbesondere etwa 1 : 3 enthalten.

4. Kosmetische Stifte, dadurch gekennzeichnet, daß sie Emulsionen darstellen und als wesentliche Bestandteile
(a) Bienenwachs,
(b) einen oder mehrere Ester, gewählt aus der Gruppe Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat,
(c) Wasser, sowie
(d) gegebenenfalls weitere Lipide und/oder übliche Hilfs- und Zusatzstoffe
enthalten.

5. Kosmetische Stifte nach einem der Ansprüche 1 - 4, dadurch gekennzeichnet, daß sie einen oder mehrere Emulgatoren aus der Gruppe der Polyglycerinfettsäureester, insbesondere das Polyglyceryl-3-diisostearat, enthalten.

6. Kosmetische Stifte nach Anspruche 4, dadurch gekennzeichnet, daß sie neben den Komponenten (a), (b) und (c) zusätzlich Fettkomponenten, gewählt aus der Gruppe
(d) Glycerintricarbonsäureester
(e) Guerbet-Alkohole
(f) Myristylmyristat
(g) Jojobaöl
(h) fraktionierte Kokosöle,
enthalten.

7. Kosmetische Stifte nach Anspruch 6, dadurch gekennzeichnet, daß sie neben den Komponenten (a) - (h) zusätzlich Stoffe, gewählt aus der Gruppe
Wachse, Kohlenwasserstoffe, Fette, Öle, Hilfs- und Zusatzstoffe wie Parfümöle, Konservierungsmittel, Farbpigmente, Lichtschutzmittel, Stabilisatoren sowie der fettlöslichen und/oder wasserlöslichen Wirkstoffe
enthalten.

8. Verfahren zur Herstellung von kosmetischen Stiften nach einem der Ansprüche 1 - 7, dadurch gekennzeichnet, daß man
(1)
(a) Bienenwachs und
(b) einen oder mehrere Ester aus
(ba) einer gesättigten Carbonsäure mit 14 - 34 Kohlenstoffatomen und
(bb) einem gesättigten Alkohol mit 20 - 40 Kohlenstoffatomen
beziehungsweise
einen oder mehrere Ester, gewählt aus der Gruppe Hexacosanylpalmitat, Octaconsanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat,
sowie gegebenenfalls einen oder mehrere Emulgatoren, gewählt aus der Gruppe der W/O-Emulgatoren, insbesondere der Polyglycerinfettsäureester, besonders vorteilhaft: Polyglyceryl-3-diisostearat
sowie Fettkomponenten, gewählt aus der Gruppe der Glycerintricarbonsäureester, Guerbet-Alkohole, Myristylmyristat, Jojobaöl und/oder verwandter Substanzen und der fraktionierten Kokosöle
aufschmilzt und
(2) kontinuierlich Wasser hinzugibt, bis der gewünschte Wassergehalt des kosmetischen Stiftes erreicht ist,
(3) während der Wasserzugabe die entstandene Mischung verrührt,
(4) und die gleichmäßige verrührte Mischung dann in Gußformen einbringt und langsam abkühlen läßt.

9. Verwendung kosmetischer Stifte nach einem der Ansprüche 1 - 7 als Lippenstifte, bevorzugt Lippenpflegestifte.

## Claims

1. Cosmetic sticks characterized in that they represent emulstions and comprise, as essential components,
(a) beeswax,
(b) one or more esters of
(ba) a saturated carboxylic acid having 14-34 carbon atoms and
(bb) a saturated alcohol having 20-40 carbon atoms,
(c) water and
(d) optionally other lipids and/or customary auxiliaries and additives.

2. Cosmetic sticks according to Claim 1, characterized in that they contain
0.5 - 50% by weight of beeswax,
0.5 - 50% by weight of one or more esters of a saturated carboxylic acid having 14-34 carbon atoms and a saturated alcohol having 20-40 carbon atoms, and
0.1 - 20% by weight of water,
in particular
2.0 - 20% by weight of beeswax,
2.0 - 25% by weight of one or more esters of a saturated carboxylic acid having 14-34 carbon atoms and a saturated alcohol having 20-40 carbon atoms, and
1.0 - 10% by weight of water,
preferably
3.0 - 10% by weight of beeswax;
5.0 - 20% by weight of one or more esters of a saturated carboxylic acid having 14-34 carbon atoms and a saturated alcohol having 20-40 carbon atoms, and
1.0 - 5% by weight of water,
very particularly preferably
4.0 - 6.0% by weight of beeswax,
15.0 - 18.0% by weight of one or more esters of a saturated carboxylic acid having 14-34 carbon atoms and a saturated alcohol having 20-40 carbon atoms, and
2.0 - 3.0% by weight of water.

3. Cosmetic sticks according to Claim 1 or 2, characterized in that they contain beeswax and one or more esters of a saturated carboxylic acid having 14-34 carbon atoms and a saturated alcohol having 20-40 carbon atoms in the ratio of 1:1 to 1:5, in particular approximately 1:3.

4. Cosmetic sticks, characterized in that they represent emulsions and comprise, as essential components,
(a) beeswax,
(b) one or more esters selected from the group consisting of hexacosanyl palmitate, octacosanyl palmitate, triacontanyl palmitate, dotriacontanyl palmitate, tetratriacontanyl palmitate, hexacosanyl stearate, octacosanyl stearate, triacontanyl stearate, dotriacontanyl stearate, tetratriacontanyl stearate,
(c) water, and
(d) optionally other lipids and/or customary auxiliaries and additives.

5. Cosmetic sticks according to one of Claims 1-4, characterized in that they contain one or more emulsifiers from amongst the group of the polyglycerol fatty acid esters, in particular polyglyceryl.

6. Cosmetic sticks according to Claim 4, characterized in that they additionally contain, besides the components (a), (b) and (c), fatty components selected from amongst the group comprising
(d) glycerol tricarboxylic acid esters,
(e) Guerbet alcohols,
(f) myristyl myristate,
(g) jojoba oil and
(h) fractionated coconut oils.

7. Cosmetic sticks according to Claim 6, characterized in that they additionally contain, besides the components (a) - (h), substances selected from amongst the group comprising waxes, hydrocarbons, fats, oils, auxiliaries and additives, such as perfume oils, preservatives, pigments, light stabilizers, stabilizers and fat- and/or water-soluble active substances.

8. Process for the preparation of cosmetic sticks according to one of Claims 1-7, characterized in that
(1)
(a) beeswax and
(b) one or more esters of
(ba) a saturated carboxylic acid having 14-34 carbon atoms and
(bb) a saturated alcohol having 20-40 carbon atoms,
or
one or more esters selected from the group consisting of hexacosanyl palmitate, octacosanyl palmitate, triacontanyl palmitate, dotriacontanyl palmitate, tetratriacontanyl palmitate, hexacosanyl stearate, octacosanyl stearate, triacontanyl stearate, dotriacontanyl stearate, tetratriacontanyl stearate
and, if appropriate, one or more emulsifiers selected from amongst the group of the W/O emulsifiers, in particular the polyglycerol fatty acid esters, particularly advantageously polyglyceryl 3-diisostearate,
and fatty components selected from amongst the group of the glycerol tricarboxylic acid esters, Guerbet alcohols, myristyl myristate, jojoba oil and/or related substances and the fractionated coconut oils are melted and
(2) water is added continuously until the desired water content of the cosmetic stick is achieved,
(3) the resulting mixture is stirred during the addition of water, and
(4) the uniform, stirred mixture is then introduced into moulds and allowed to cool slowly.

9. Use of cosmetic sticks according to one of Claims 1-7 as lipsticks, preferably lip care sticks.

## Revendications

1. Bâtons cosmétiques, caractérisés en ce qu'ils représentent des émulsions et contiennent, en tant que composants essentiels
(a) de la cire d'abeille,
(b) un ou plusieurs esters de
(ba) un acide carboxylique saturé ayant de 14 à 34 atomes de carbone et
(bb) un alcool saturé ayant de 20 à 40 atomes de carbone,
(c) de l'eau, ainsi que
(d) éventuellement d'autres lipides et/ou des additifs et adjuvants usuels.

2. Bâtons cosmétiques selon la revendication 1, caractérisés en ce qu'ils contiennent
0,5 - 50 % en poids de cire d'abeille
0,5 - 50 % en poids d'un ou plusieurs esters d'un acide carboxylique saturé ayant de 14 à 34 atomes de carbone et d'un alcool saturé ayant de 20 à 40 atomes de carbone,
0,1 - 20 % en poids d'eau,
en particulier
2,0 - 20 % en poids de cire d'abeille,
2,0 - 25 % en poids d'un ou plusieurs esters d'un acide carboxylique saturé ayant de 14 à 34 atomes de carbone et d'un alcool saturé ayant de 20 à 40 atomes de carbone,
1,0 - 10 % en poids d'eau,
de préférence
3,0 - 10 % en poids de cire d'abeille,
5,0 - 20 % en poids d'un ou plusieurs esters d'un acide carboxylique saturé ayant de 14 à 34 atomes de carbone et d'un alcool saturé ayant de 20 à 40 atomes de carbone,
1,0 - 5 % en poids d'eau,
et de façon particulièrement préférée,
4,0 - 6,0 % en poids de cire d'abeille,
15,0 - 18,0 % en poids d'un ou plusieurs esters d'un acide carboxylique saturé ayant de 14 à 34 atomes de carbone et d'un alcool saturé ayant de 20 à 40 atomes de carbone,
2,0 - 3,0 % en poids d'eau.

3. Bâtons cosmétiques selon la revendication 1 ou 2, caractérisés en ce qu'ils contiennent de la cire d'abeille et un ou plusieurs esters d'un acide carboxylique saturé ayant de 14 à 34 atomes de carbone et d'un alcool saturé ayant de 20 à 40 atomes de carbone, dans un rapport allant de 1:1 à 1:5, en particulier d'environ 1:3.

4. Bâtons cosmétiques, caractérisés en ce qu'ils représentent des émulsions et contiennent, en tant que composants essentiels
(a) de la cire d'abeille,
(b) un ou plusieurs esters, choisis parmi le palmitate d'hexacosanyle, le palmitate d'octacosanyle, le palmitate de triacontanyle, le palmitate de dotriacontanyle, le palmitate de tétratriacontanyle, le stéarate d'hexacosanyle, le stéarate d'octacosanyle, le stéarate de triacontanyle, le stéarate de dotriacontanyle, le stéarate de tétratriacontanyle,
(c) de l'eau, ainsi que
(d) éventuellement d'autres lipides et/ou additifs et adjuvants usuels.

5. Bâtons cosmétiques selon l'une des revendications 1 à 4, caractérisés en ce qu'ils contiennent un ou plusieurs émulsifiants choisis parmi des polyesters d'acides gras et de glycérol, en particulier le 3-diisostéarate de polyglycéryle.

6. Bâtons cosmétiques selon la revendication 4, caractérisés en ce qu'ils contiennent, en plus des composants (a), (b) et (c), des composants gras supplémentaires choisis parmi
(d) les tricarboxylates de glycérol,
(e) les alcools de Guerbet,
(f) le myristate de myristyle,
(g) l'huile de jojoba,
(h) des huiles de coprah fractionnées.

7. Bâtons cosmétiques selon la revendication 6, caractérisés en ce qu'ils contiennent, en plus de composants (a) - (h), des substances supplémentaires choisies parmi des cires, des hydrocarbures, des graisses, des huiles, des adjuvants et additifs tels que des huiles essentielles, conservateurs, pigments colorés, agents de protection contre la lumière, stabilisants, ainsi que les substances actives liposolubles et/ou hydrosolubles.

8. Procédé pour la fabrication de bâtons cosmétiques selon l'une des revendications 1 à 7, caractérisé en ce que
(1) on fait fondre
(a) de la cire d'abeille et
(b) un ou plusieurs esters de
(ba) un acide carboxylique saturé ayant de 14 à 34 atomes de carbone et
(bb) un alcool saturé ayant de 20 à 40 atomes de carbone,
ou
un ou plusieurs esters choisis parmi le palmitate d'hexacosanyle, le palmitate d'octacosanyle, le palmitate de triacontanyle, le palmitate de dotriacontanyle, le palmitate de tétratriacontanyle, le stéarate d'hexacosanyle, le stéarate d'octacosanyle, le stéarate de triacontanyle, le stéarate de dotriacontanyle, le stéarate de tétratriacontanyle,
ainsi qu'éventuellement un ou plusieurs émulsifiants, choisis dans le groupe des émulsifiants E/H, en particulier des polyesters d'acides gras et de glycérol, de façon particulièrement avantageuse le 3-diisostéarate de polyglycéryle, ainsi que des composants gras choisis parmi les tricarboxylates de glycéryle, les alcools de Guerbet, le myristate de myristyle, l'huile de jojoba et/ou des substances apparentées et les huiles de coprah fractionnées, et
(2) on y ajoute en continu de l'eau jusqu'à ce que soit atteinte la teneur désirée en eau du bâton cosmétique,
(3) on délaie pendant l'addition d'eau le mélange résultant,
(4) et on introduit ensuite dans des moules le mélange délayé de façon homogène, puis on le laisse refroidir lentement.

9. Utilisation de bâtons cosmétiques selon l'une des revendications 1 à 7, en tant que bâtons pour les lèvres, de préférence bâtons de soins labiaux.
